(19) Europäisches Patentamt
European Patent Office
Office européen des brevets

(11) Numéro de publication: **0 023 849**
**B1**

(12) # FASCICULE DE BREVET EUROPEEN

(45) Date de publication du fascicule du brevet: **04.05.83**

(51) Int. Cl.³: **C 07 C 59/74**, C 07 D 307/93, C 07 D 307/32

(21) Numéro de dépôt: **80400789.6**

(22) Date de dépôt: **03.06.80**

(54) **Acide penténoique substitué, procédé pour sa préparation, son application à la préparation de dérivés cyclopropaniques substitués.**

(30) Priorité: **06.06.79 FR 7914425**

(43) Date de publication de la demande:
**11.02.81 Bulletin 81/6**

(45) Mention de la délivrance du brevet:
**04.05.83 Bulletin 83/18**

(84) Etats contractants désignés:
**AT BE CH DE GB IT LI LU NL SE**

(56) Documents cités:
**FR-A-1 350 741**

(73) Titulaire: **ROUSSEL-UCLAF, 35, boulevard des Invalides, F-75007 Paris (FR)**

(72) Inventeur: **Martel, Jacques, 15, rue Douvillez, F-93140 Bondy (FR)**
Inventeur: **Tessier, Jean, 30, rue Jean Moulin, F-94700 Maisons Alfort (FR)**
Inventeur: **Demoute, Jean-Pierre, 249 bis rue de Rosny, F-93100 Montreuil S/Bois (FR)**

(74) Mandataire: **Niel, Michel et al, ROUSSEL-UCLAF boîte postale no 9 102, route de Noisy, F-93230 Romainville (FR)**

EP 0 023 849 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Acide penténoïque substitué, procédé pour sa préparation, son application à la préparation de dérivés cyclopropaniques substitués

Le brevet français 1 350 741 décrit des acides comportant une fonction aldéhyde. Mais ces acides comportent 2 atomes de carbone entre le carbone porteur de la fonction aldéhyde et celui porteur de la fonction carboxyle. On décrit maintenant un composé qui ne comprend qu'un seul atome de carbone entre le carbone porteur de la fonction aldéhyde et celui de la fonction carboxyle.

La présente invention concerne un acide penténoique substitué, son procédé de préparation et son application à la préparation de dérivés cyclopropaniques substitués.

L'invention a tout d'abord pour objet l'acide 3-formyl 4-méthyl pent -3-èn 1-oique de formule I:

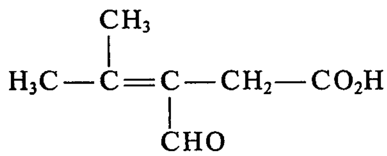
(I)

L'invention a également pour objet un procédé de préparation de l'acide 3-formyl 4-méthyl pent-3-èn 1-oïque, de formule I, caractérisé en ce que l'on fait réagir un dérivé de formule II:

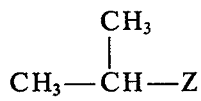
(II)

dans laquelle Z représente un groupement électroattracteur, avec un dérivé de formule III:

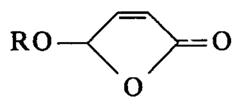
(III)

dans laquelle R représente le reste R d'un alcool ROH, puis soumet le composé résultant de formule IV:

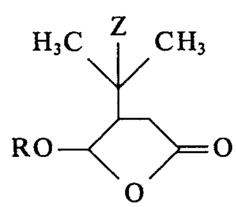
(IV)

à l'action d'un agent basique.

L'invention a plus précisément pour objet un procédé tel que décrit précédemment, caractérisé en ce que le groupement Z est un groupement $NO_2$ et en ce que l'on fait réagir les composés II et III en présence d'une base choisie dans le groupe constitué par les amines secondaires, les amines tertiaires, les hydroxydes d'ammonium quaternaires et les carbonates alcalins.

Comme amine tertiaire en présence de laquelle on fait réagir les composés II et III on utilise avantageusement la triéthylamine.

L'invention a également pour objet un procédé tel que décrit précédemment, caractérisé en ce que le groupement Z est un groupement hydroxyle et en ce que l'on fait réagir les composés II et III sous irradiation ou en présence d'un promoteur radicalaire.

Comme promoteur radicalaire on utilise avantageusement le peroxyde de benzoyle, l'azo bis iso butyronitrile, le peroxyde de diterbutyle, le perbenzoate de terbutyle, le peroxyde de dilauryle.

L'invention a également pour objet un procédé tel que décrit précédemment, caractérisé en ce que le groupement Z est un groupement aryl sulfonyle et en ce que l'on fait réagir les composés II et III en présence d'une base forte au sein d'un solvant organique.

Le radical aryle du groupement aryl sulfonyle est notamment un radical paratolyle.

L'invention a également pour objet un procédé tel que décrit précédemment, caractérisé en ce que le groupement Z est un groupement halogéno triaryl phosphonio et en ce que l'on fait réagir les composés II et III en présence d'une base forte au sein d'un solvant organique.

Le groupement halogéno triaryl phosphonic est notamment le radical iodo triphényl phosphonio.

L'invention a également pour objet un procédé de préparation tel que défini précédemment, dans lequel le groupement Z est un groupement aryl sulfonyle ou un groupement halogéno triaryl phosphonio, caractérisé en ce que la base forte est choisie dans le groupe constitué par les alkyl lithiens, les amidures alcalins, les hydrures alcalins, les alcoolates alcalins, et en ce que le solvant utilisé est choisi dans le groupe constitué par le tétrahydrofurane, le diméthylsulfoxyde, le

diméthoxyéthane, le diméthylformamide, l'hexaméthylphosphoramide, les hydrocarbures aromatiques et des cycloalcanes.

Lorsque Z est un groupement aryl sulfonyle ou un groupement halogéno triaryl phosphonio, la réaction entre les composés II et III est effectuée avantageusement en présence de butyllithium au sein d'un mélange de tétrahydrofurane et de cyclohexane.

Selon le procédé de l'invention, l'agent basique que l'on fait réagir sur les composés IV est notamment le carbonate de sodium utilisé en milieu hydrométhanolique.

L'invention a également pour objet l'application de l'acide 3-formyl 4-méthyl pent-3-èn 1-oïque de formule I à la préparation de composés de formule V:

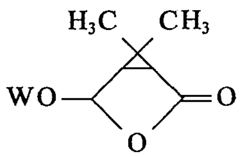

(V)

dans laquelle W représente un atome d'hydrogène ou un reste $R_1$ d'un alcool $R_1OH$ éventuellement chiral, caractérisée en ce que l'on fait réagir en milieu anhydre l'acide 3-formyl 4-méthyl pent-3-èn 1-oïque avec un hydracide HX, dans lequel X représente un atome d'halogène, en présence d'un halogénure de lithium LiX, au sein d'un solvant organique, pour obtenir un composé de formule VI:

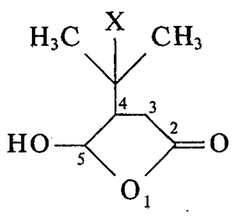

(VI)

que l'on fait réagir soit avec un alcool $R_1OH$ achiral pour obtenir le composé VII racémique:

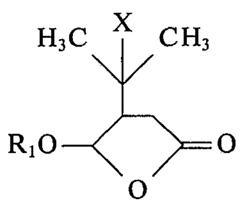

(VII)

qui, comme de dérivé VI, présente une relation trans entre les substituants en 4 et 5 de la tétrahydrofuranone, soit avec un alcool $R_1OH$ chiral, le composé VII étant alors obtenu sous forme d'un mélange des deux diastéréoisomères prévisibles, mélange que l'on peut séparer en ses constituants par des procédés physiques, fait réagir le composé de formule VII, sous forme racémique lorsque $R_1$ est achiral, ou sous forme optiquement active lorsque $R_1$ est chiral, le composé VII étant, dans ce dernier cas, à l'état de l'un ou de l'autre des diastéréoisomères, avec un agent basique, pour obtenir le composé bicyclique de formule V':

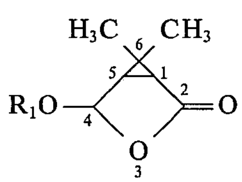

(V')

sous forme racémique, ou sous l'une ou l'autre de ses formes énantiomériques selon que $R_1$ est respectivement un reste achiral ou chiral, la configuration absolue en position 4 de ce composé ainsi que celles qui en découlent en positions 1 et 5 étant alors déterminées par la stéréochimie du diastéréoisomère VII mis en oeuvre, puis hydrolyse, si désiré, l'éther de formule V', en milieu acide, avec rétention totale des configurations absolues, pour obtenir le composé de formule $V_A$ correspondant:

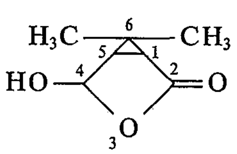

($V_A$)

L'invention a plus particulièrement pour objet l'application telle que définie précédemment, caractérisée en ce que la transformation du composé de formule I en composé de formule VI est effectuée à l'aide des couples BrH/BrLi ou ClH/ClLi, au sein de l'éther éthylique anhydre, ainsi que l'application telle que définie précédemment, caractérisée en ce que l'on fait réagir l'alcool $R_1OH$, achiral ou chiral et le composé VI en présence d'acide paratoluène sulfonique.

Selon l'application de l'invention, les deux diastéréoisomères provenant de l'action de l'alcool chiral $R_1OH$ sur le racémate de composé VI, sont séparés par chromatographie ou bien par cristallisation.

Selon un mode d'application avantageux de l'invention, la transformation du composé VII en composé cyclopropanique V' est effectué à l'aide du butyllithium.

Selon l'application de l'invention, on effectue aussi avantageusement la transformation des tétrahydrofuranones VII en composé cyclopropanique. V' soit par la méthode de catalyse en transfert de phase, à l'aide d'un hydroxyde alcalin, d'eau, d'un solvant non miscible à l'eau et d'un ammonium quaternaire, soit à l'aide d'hydrure de sodium.

Dans la méthode de la catalyse par transfert de phase on utilise avantageusement l'hydroxyde de sodium.

L'hydrolyse des composés V' en composés $V_A$ est effectuée en présence d'acide chlorhydrique, avantageusement en milieu hydroacétonique.

Les produits industriels nouveaux qui suivent sont utiles notamment dans la mise en oeuvre du procédé de l'invention:

a) les composés de formule générale IV:

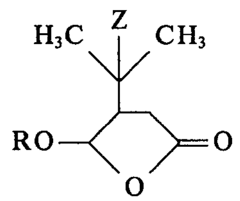

(IV)

dans laquelle Z représente un groupement électroattracteur et notamment un groupement hydroxy, un groupement nitro, un groupement paratolylsulfonyle ou un groupement halogéno triphényl phosphonio et R représente le reste d'un alcool R—OH,

b) les composés de formule générale VIII:

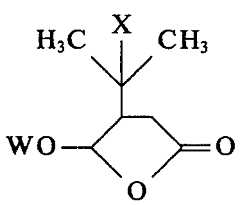

(VIII)

dans laquelle X représente un atome d'halogène et notamment un atome de brome ou de chore et W représente de l'hydrogène ou un radical $R_1$, $R_1$ représentant le reste d'un alcool $R_1-OH$ éventuellement chiral.

Il existait déjà des procédés d'hémisynthèse permettant de préparer la 6,6-diméthyl 4-hydroxy 3-oxabicyclo [3.1.0] hexan-2-one, au départ d'un produit très élaboré: l'acide chrysanthémique (voir par exemple le brevet français 1580474).

La société demanderesse a maintenant mis au point, par l'intermédiaire du composé I, un procédé de synthèse totale de la 6,6-diméthyl 4-hydroxy 3-oxabicyclo [3.1.0] hexan-2-one ou de ses éthers, sous forme racémique ou optiquement active. Ce procédé utilise des réactifs facilement accessibles. Ses étapes sont en nombre limité.

L'accès par synthèse totale à un composé tel que la 6,6-diméthyl 4-hydroxy 3-oxabicyclo [3.1.0] hexan-2-one qui sert d'intermédiaire dans la synthèse de nombreux esters insecticides d'activité élevée (voir brevet français 2185612), présente un intérêt particulièrement remarquable.

Les exemples suivants illustrent l'invention sans la limiter.

Exemple 1:

Acide 3-formyl 4-méthyl pent-3-èn 1-oique

Stade A:

4-(2-nitro prop-2-yl) 5-méthoxy tétrahydrofuran 2-one trans dl

On agite pendant 70 heures, à 20/25° C, 9 $cm^3$ de 2-nitropropane 7 g de 5-méthoxy 2,5-dihydrofuran 2-one 3-ène et 1 $cm^3$ de triéthylamine, lave le mélange réactionnel avec une solution aqueuse de phosphate monosodique, extrait au benzène, sèche, concentre à sec sous pression réduite, reprend le résidu par de l'éther isopropylique, amorce la cristallisation et refroidit à 0° C sous agitation, essore et obtient 8,42 g de produit cristallisé F$\simeq$33° C.

Analyse: $C_8H_{13}NO_5=203,18$

| | | | |
|---|---|---|---|
| Calculé: | C 47,29 | H 6,45 | N 6,89% |
| Trouvé: | C 47,10 | H 6,50 | N 6,70% |

Spectre de RMN

pic à 1,6 ppm attribué aux hydrogènes des méthyles géminés pics à 2,0—3,17 ppm attribués aux hydrogènes en 3 et 4 du cyclopentyle

pics à 5,25—5,28 ppm attribués à l'hydrogène en 5 du cyclopentyle

pic à 3,5 ppm attribué aux hydrogènes du méthoxy

Stade B:

Acide 3-formyl 4-méthyl pent-3-èn 1-oïque

On dissout 18,9 g de carbonate de sodium dans 180 $cm^3$ d'eau, refroidit à 0°C, ajoute, en une seule fois, 18,2 g de la nitrolactone obtenue précédemment et dissoute dans 25 $cm^3$ de méthanol. Après 120 heures à température ambiante, on lave le mélange réactionnel à l'éther éthylique, refroidit à 0°C, ajoute avec précaution et sous atmosphère inerte, de l'acide sulfurique concentré jusqu'à pH$\simeq$1, extrait au chloroforme puis à l'acétate d'éthyle, sèche les phases organiques réunies, les concentre à sec sous pression réduite, pour obtenir 9,5 g de produit brut qui, par cristallisation dans l'eau, donnent 7,4 g de produit pur F=102° C.

Analyse: $C_7H_{10}O_3=142,156$

| | | |
|---|---|---|
| Calculé: | C 59,14 | H 7,09% |
| Trouvé: | C 59,20 | H 7,0% |

Spectre de RMN

pic à 20 ppm attribué aux hydrogènes en 5

pic à 2,26 ppm attribué aux hydrogènes du méthyle en 4

pic à 3,38 ppm attribué aux hydrogènes en 2

pic à 10,13 ppm attribué à l'hydrogène du formyle

Exemple II:

Acide 3-formyl 4-méthyl pent-3-èn 1-oïque

Stade A:

4-(2-hydroxy prop-2-yl) 5-(3-phénoxyphényl) méthoxy tétrahydrofuran 2-one -trans dl-

On chauffe au reflux, sous agitation et sous atmosphère inerte, 3,5 g de 5-(3-phénoxyphényl) méthoxy 2,5-dihydrofuran-2-one dans 100 $cm^3$ d'isopropanol, ajoute régulièrement en une heure et demie par portions de 25 mg, 300 mg de peroxyde de benzoyle, concentre à sec sous pression réduite à 40—50° C, chromatographie le résidu sur silice puis élue avec un mélange benzène-acétate d'éthyle 8-2, recueille 3,7 g de produit brut qui, repris par l'éther isopropylique, donne des cristaux blancs F=50—55° C.

Analyse: $C_{20}H_{22}O_3=342,39$

| | | |
|---|---|---|
| Calculé: | C 70,16 | H 6,4% |
| Trouvé: | C 69,9 | H 6,5% |

Spectre de RMN (ppm)
pics à 1,18 et 1,21 attribués aux hydrogènes des méthyles
pics à 2,16—2,75 attribués aux hydrogènes en 3 et 4 du cyclopentyle
pics à 4,48—4,35 et 4,8—5 attribués aux hydrogènes du méthylène benzylique
pics à 5,59—5,58 attribués aux hydrogènes en 5 du cyclopentyle
pics à 6,83—7,5 attribués aux hydrogènes des noyaux aromatiques
pic à 1,5 attribué à l'hydrogène de l'hydroxyle

La 5-(3-phénoxyphényl) méthoxy 2,5-dihydrofuran-2-one utilisée au départ de l'exemple a été préparée comme suit: On mélange 12 g de 5 hydroxy 2-(5H) furanone, 250 $cm^3$ de benzène, 25 g d'alcool métaphénoxy benzylique et 200 mg d'acide paratoluène sulfonique, agite en distillant du benzène en le remplaçant plusieurs fois par la même quantité de benzène sec, dans le mélange réactionnel, après deux heures, laisse refroidir à température ambiante et lave avec une solution saturée de bicarbonate de sodium puis à l'eau, sèche, concentre à sec sous pression résuite, obtient 39,7 g d'une huile que l'on purifie par chromatographie sur silice en éluant par un mélange benzène — acétate d'éthyle 9-1 et recueille 24,9 g du produit attendu.

Spectre de RMN
pics à 7,3—7,4 ppm attribués aux hydrogènes en 4 de la furanone
pics à 6,18—6,32 ppm attribués aux hydrogènes en 3 de la furanone
pic à 6,03 ppm attribué à l'hydrogène en 5 de la furanone
pics à 4,58—4,76 et 4,85—5,12 ppm attribués aux hydrogènes du méthoxy
pics à 6,92—7,5 ppm attribués aux noyaux aromatiques

Stade B:

Acide 3-formyl 4-méthyl pent-3-èn 1-oïque

On dissout 619 mg de carbonate de sodium dans 6 $cm^3$ d'eau, refroidit à 0° C, ajoute en agitant 1 g d'hydroxylactone obtenue précédemment et 2 $cm^3$ de méthanol, après 19 heures de contact à température ambiante, lave à l'éther éthylique, refroidit à 0° C, ajoute lentement de l'acide sulfurique concentré jusqu'à pH ~ 1, extrait au méthanol et au chloroforme, réunit les phases organiques, les sèche, les concentre à sec sous pression réduite, obtient 245 mg de produit brut qui, recristallisé, donne un produit fondant à 102° C.

Exemple III:

Acide 3-formyl 4-méthyl pent-3-èn 1-oïque

Stade A:

4-(2-paratoluène sulfonyl prop-2-yl) 5-méthoxy tétrahydrofuran 2-one, trans dl

On mélange 500 mg d'isopropyl paratolyl sulfone et 10 $cm^3$ de tétrahydrofurane sous agitation et sous atmosphère inerte, refroidit à −70° C, ajoute lentement 1,3 $cm^3$ d'une solution cyclohexanique 1,95 M de butyl lithium, agite encore 1/2 heure à −70° C, ajoute alors, en 10 minutes, 287 mg de 5-méthoxy 2,5-dihydrofuran 2-one en solution dans 4 $cm^3$ de tétrahydrofurane, maintient penfant une heure à −70° C, verse le mélange réactionnel sur une solution aqueuse de phosphate monosodique à 0° C, extrait au chlorure de méthylène, lave à l'eau, sèche, concentre à sec sous pression réduite, cristallise le résidu dans l'éther isopropylique et obtient 490 mg de cristaux blancs F = 129° C.

Analyse:

| | | | |
|---|---|---|---|
| Calculé: | C 57,67 | H 6,45 | S 10,26% |
| Trouvé: | C 57,6 | H 6,5 | S 10,1% |

Spectre de RMN
pics à 1,27—1,32 ppm attribués aux hydrogènes des méthyles géminés
pic à 2,46 ppm attribué aux hydrogènes du méthyle porté par l'aromatique
pic à 2,75 ppm attribué aux hydrogènes 3 et 4 du cyclopentyle
pic à 5,58 ppm attribué à l'hydrogène du cyclopentyle an 5
pic à 3,51 ppm attribué aux hydrogènes du méthoxy
pics à 7,28—7,42 ppm attribués aux hydrogènes en 3 et 5 de l'aromatique
pics à 7,66—7,8 ppm attribués aux hydrogènes en 2 et 6 de l'aromatique

Stade B:

Acide 3-formyl 4-méthyl pent-3-èn 1-oïque

On agite 250 mg du produit obtenu précédemment, 4 cm³ d'eau, 0,8 cm³ de méthanol et 250 mg de carbonate de sodium pendant 2 jours à température ambiante. Après lavage à l'éther, on acidifie la phase aqueuse à pH 3—3,5, avec de l'acide chlorhydrique N, sature de chlorure de sodium, extrait au chloroforme, sèche, concentre à sec sous pression réduite, et obtient 87 mg du produit attendu F = 102° C.

Ce produit est identique en tous points à l'acide 3-formyl 4-méthyl pent-3-èn 1-oïque obtenu par une autre voie.

Exemple IV:

Acide 3-formyl 4-méthyl pent-3-èn 1-oïque

Stade A:

Iodure de [1(5-méthoxy 2-oxo 4,5-dihydro 3H furan-4-yl)-1-méthyl 1-éthyl] triphényl phosphonium

a) Préparation de l'ylure

A une suspension de 3 g d'iodure de triphényl isopropylphosphonium dans 40 cm³ de tétrahydrofurane on ajoute en une fois 3,5 cm³ de solution 2M de butyllithium dans le cyclohexane, agite pendant 10 minutes à température ambiante et obtient une solution d'ylure (solution A).

b) Addition de l'ylure sur la furanone

La solution A refroidie à −60° C est ajoutée lentement sous atmosphère inerte, à une solution de 0,800 g de 5-méthoxy 2,5-dihydrofuran 2-one 3-ène dans 50 cm³ de tétrahydrofurane également refroidie à −60° C. On agite pendant 10 minutes, verse le mélange réactionnel sur un mélange de solution aqueuse de phosphate monosodique et de glace, lave à l'éther, extrait au chlorure de méthylène, sèche, concentre à sec sous pression réduite et obtient 3,4 g de sel de phosphonium, fondant vers 140° C.

Analyse: $C_{26}H_8IO_3P = 546,37$

| | | | | |
|---|---|---|---|---|
| Calculé: | C 57,15 | H 5,16 | I 23,24 | P 5,67% |
| Trouvé: | C 57,3 | H 5,2 | I 22,4 | P 5,3% |

Spectre de RMN

pics à 1,63—1,80—1,95—2,10 ppm attribués aux hydrogènes des méthyles an $\beta$ du phosphore

pics à 2,22—3,33 ppm attribués aux hydrogènes portés par les carbones en position 3 et 4 de la furanone

pic à 3,23 ppm attribué aux hydrogènes du méthoxy

pics à 5,46—5,54 ppm attribués à l'hydrogène en 5 de la furanone

pics à 7,81—7,92 ppm attribués aux hydrogènes des noyaux aromatiques

Stade B:

Acide 3-formyl 4-méthyl pent-3-ène 1-oïque

A une solution de 0,700 g d'iodure de [1(5-méthoxy 2-oxo 4,5-dihydro 3H furan-4-yl)-1-méthyl 1-éthyl] triphényl phosphonium dans 1 cm³ de méthanol on ajoute 0,700 g de carbonate de sodium en solution dans 8 cm³ d'eau, agite pendant 2 heures à 20° C, élimine par filtration l'insoluble formé, acidifie le filtrat à pH 2, sature par du chlorure de sodium, extrait au chloroforme, concentre à sec, purifie dans l'éther isopropylique et obtient 0,095 g d'acide 3-formyl 4-méthyl pent-3-ène 1-oïque F = 102° C.

Exemple V:

6,6-diméthyl 4-hydroxy 3-oxa bicyclo [3.1.0] hexan 2-one (dl)

Stade A:

4-(2-chloro-prop-2-yl) 5-hydroxy tétrahydrofuran 2-one dl trans

Sous un courant d'acide chlorhydrique anhydre, on agite 1 g d'acide 3-formyl 4-méthyl pent-3-ène 1-oïque obtenu à l'exemple 1, 2, 3 ou 4, $cm^3$ d'éther éthylique anhydre et 1 g de chlorure de lithium sec pendant 2 heures à −30°C, puis 2 heures à 0°C, arrête le courant d'acide chlorhydrique et continue l'agitation 48 heures à température ambiante, après 54 heures de contact, verse le mélange réactionnel dans de l'eau glacée, décante, extrait au benzène, sèche, concentre à sec sous pression réduite, obtient 1,1 g de produit brut, le chromatographie sur silice en éluant par un mélange benzène — acétate d'éthyle 1-1, récupère 545 mg d'un produit qui cristallise F = 80°C.

Analyse: $C_7H_{11}ClO_3 = 178,617$

| | | | |
|---|---|---|---|
| Calculé: | C 47,07 | H 6,21 | Cl 19,85% |
| Trouvé: | C 47,2 | H 6,2 | Cl 19,5% |

Spectre de RMN

pics à 1,55 et 1,66 ppm attribués aux hydrogènes des méthyles
pics à 2,42—2,92 ppm attribués aux hydrogènes en position 3 et 4 du cycle
pics à 5,82—5,89 ppm attribués à l'hydrogène en 5 du cycle
pic à 3,83 ppm attribué à l'hydrogène de l'hydroxyle

Stade B:

4-(2-chloro-prop-2-yl) 5-[(3-phénoxyphényl)méthoxy)] tétrahydrofuran 2-one

On agite pendant 19 heures à température ambiante 393 mg du produit obtenu précédemment, 668 mg d'alcool métaphénoxybenzylique, 20 mg d'acide paratoluènesulfonique et 5 $cm^3$ de benzène, neutralise avec un peu de bicarbonate de sodium, sèche, concentre à sec sous pression réduite, obtient 1,18 g de produit brut, le chromatographie sur silice en éluant par du benzène, récupère 467 mg de produit, qui, recristallisé dans l'éther de pétrole, fond près de 50°C.

Analyse: $C_{20}H_{21}ClO_4 = 360,84$

| | | | |
|---|---|---|---|
| Calculé: | C 66,57 | H 5,87 | Cl 9,83% |
| Trouvé: | C 66,60 | H 5,80 | Cl 9,90% |

Spectre de RMN

pics à 1,5 et 1,55 ppm attribués aux hydrogènes des méthyles
pics à 2,55—2,72 ppm attribués aux hydrogènes en position 3 et 4 du cyclopentyle
pics à 5,52—5,56 ppm attribués à l'hydrogène en position 5 du cyclopentyle
pics à 6,92—7,5 ppm attribués aux hydrogènes des noyaux aromatiques
pics à 4,47—4,66 et 4,77—4,97 ppm attribués aux hydrogènes du méthylène benzylique

Stade C:

dl 6,6-diméthyl 4-hydroxy 3-oxabicyclo [3.1.0] hexan 2-one

On refroidit, vers −20°C, 0,55 $cm^3$ d'une solution molaire de diisopropylamine dans du tétrahydrofurane et 5 $cm^3$ de tétrahydrofurane, ajoute 0,25 $cm^3$ d'une solution de n-butyllithium 2M dans du cyclohexane, laisse remonter la température à 0°C, puis refroidit à −60°C −70°C, ajoute en une seule fois 180 mg du produit obtenu au stade B, après 2 heures de réchauffement jusqu'à 0°C, maintient 1 heure cette température, verse le mélange réactionnel dans de l'acide chlorhydrique 2N glacé et laisse 17 heures à 20°C sous vive agitation, décante, extrait au chloroforme, sèche, concentre à sec, reprend le résidu obtenu par un mélange éther isopropylique — éther de pétrole, extrait à l'eau, concentre à sec sous pression réduite la phase aqueuse pour obtenir 30 mg de produit cristallisé F = 80°C.

Exemple VI:

6,6-diméthyl 4-hydroxy 3-oxabicyclo [3.1.0] hexan 2-one (dl)

Stade A:

4-(2-bromoprop-2-yl) 5-hydroxy tétrahydrofuran 2-one dl trans

On refroidit à −35° C un mélange de 2,35 g d'acide 3-formyl 4-méthyl pent-3-ène 1-oïque obtenu à l'exemple 1 ou 2, 2,7 g de bromure de lithium sec et 60 $cm^3$ d'éther éthylique anhydre, agite 90 minutes sous courant d'acide bromhydrique sec, chasse celui-ci par un fort courant d'azote sec tout en maintenant la température entre −30° C et −40° C, verse le mélange réactionnel dans de l'eau glacée, extrait au benzène, sèche la phase organique, concentre à sec sous pression réduite sans chauffer, récupère 3,27 g d'une huile qui cristallise, recristallise le produit dans l'éther de pétrole et isole 2,5 g de cristaux blancs fondant à ~75° C.

Analyse: $C_7H_{11}BrO_3$ = 223,073

| | | | |
|---|---|---|---|
| Calculé: | C 37,69 | H 4,97 | Br 35,82% |
| Trouvé: | C 37,80 | H 5,20 | Br 35,20% |

Spectre de RMN

pics à 1,74 et 1,86 ppm attribués aux hydrogènes des méthyles
pics à 2,25—3,0 ppm attribués aux hydrogènes en position 3 et 4 du cycle
pics à 5,87 et 5,93 ppm attribués à l'hydrogène en position 5 du cycle
pic à 3,92 ppm attribué à l'hydrogène de l'hydroxyle

Stade B:

4-(2-bromo-prop-2-yl) 5[(3-phénoxyphényl) méthoxy] tétrahydrofuran 2-one

On agite 24 heures à température ambiante 5,5 g du produit obtenu précédemment, 5,2 g d'alcool métaphénoxy benzylique, 50 $cm^3$ de benzène et 270 mg d'acide paratoluènesulfonique, lave le milieu réactionnel avec une solution aqueuse de bicarbonate de sodium, extrait au benzène, sèche, concentre à sec sous pression réduite, obtient environ 10 g d'une huile qui cristallise, recristallise ce produit dans un mélange d'éther isopropylique — éther de pétrole (7 $cm^3$—5 $cm^3$), isole 5,83 g fondant à 60° C. Une chromatographie des liqueurs mères permet de récupérer 1 g de produit de même pureté.

Analyse: $C_{20}H_{21}BrO_4$

| | | | |
|---|---|---|---|
| Calculé: | C 59,27 | H 5,22 | Br 19,72% |
| Trouvé: | C 60,10 | H 5,60 | Br 21,50% |

Spectre de RMN

pics à 1,67—1,7 ppm attribués aux hydrogènes des méthyles
pics à 2,22—2,92 ppm attribués aux hydrogènes en position 3 et 4 du cyclopentyle
pics à 5,53—5,58 ppm attribués à l'hydrogène en position 5 du cyclopentyle
pics à 4,48—4,68 et 4,78—4,98 ppm attribués aux hydrogénes du méthylène benzylique
pics à 6,92—7,58 ppm attribués aux hydrogènes des noyaux aromatiques

Stade C:

6,6-diméthyl 4-hydroxy 3-oxabicyclo [3.1.0] hexan 2-one

On refroidit, vers −20°C, 0,55 $cm^3$ d'une solution molaire de diisopropylamine dans du tétrahydrofurane diluée avec 5 $cm^3$ de tétrahydrofurane, ajoute 0,25 $cm^3$ d'une solution de n-butyllithium 2M dans le cyclohexane, laisse réchauffer le mélange vers 0°C puis refroidit à −60°C pour ajouter en une seule fois 200 mg du dérivé bromé obtenu ci-dessus, après 15 minutes de réaction à cette température on obtient une solution de 6,6-diméthyl 4-[(3-phénoxyphényl) méthoxy] 3-oxabicyclo [3.1.0] hexan 2-one dl, la verse sur de l'acide chlorhydrique 2N glacé et laisse 17 heures à 20°C sous vive agitation, décante, extrait au chloroforme, sèche, concentre à sec, obtient 170 mg de produit, le reprend par un mélange éther isopropylique — éther de pétrole, l'extrait à l'eau, concentre la phase aqueuse à sec sous pression réduite pour obtenir 50 mg de produit qui cristallise F = 80° C.

Exemple VII:

6,6-diméthyl 4-hydroxy 3-oxabicyclo [3.1.0] hexan 2-one

Stade A:

4-(2-bromoprop-2-yl) 5-hydroxy tétrahydrofuran 2-one dl trans

La préparation de ce composé est décrite au stade A de l'exemple VI.

Stade B:

4-(2-bromo-prop 2-yl) 5-[(3-phénoxyphényl) méthyl méthoxy] tétrahydrofuran-2-one (mélange de diastéréoisomères A et B de structure trans et diastéréoisomères A et B séparés)

On agite pendant 24 heures, à 20/25°C, 6,07 g de bromolactone obtenue précédemment, 5,83 g d'alcool (S) α-méthyl 3-phénoxy benzylique, 300 mg d'acide paratoluènesulfonique et 60 $cm^3$ de benzène et 10 g de deshydratant: »Actigel« coloré, lave le mélange réactionnel avec une solution aqueuse de bicarbonate de sodium, extrait au benzène, sèche, évapore à sec sous pression réduite, obtient 9,5 g de produit brut, chromatographie le produit brut sur silice en éluant au chlorure de méthylène. Des 5 g du mélange pur des diastéréoisomères ainsi obtenu, on isole 960 mg d'isomère B par cristallisation dans l'éther isopropylique à 30% d'éther de pétrole F≃76°C $(\alpha)_D = +188°5$ (1% benzène), la chromatographie sur silice des liqueurs mères, avec élution au chlorure de méthylène, a permis d'obtenir l'isomère A pur sous forme d'une huile.

Stade C:

(1R, 5S) 6,6-diméthyl 4(R)[(S) (3-phénoxyphényl) méthyl méthoxy] 3-oxabicyclo [3.1.0] hexan-2-one

Pendant 2 heures 30 minutes à température ambiante, on agite 300 mg de dérivé bromé cristallisé (isomère B) obtenu précédemment, 3 $cm^3$ de chlorure de méthylène, 3 $cm^3$ d'une solution aqueuse d'hydroxyde de sodium à 50% ($P/_p$) et environ 30 mg de chlorure de triéthylbenzylammonium, verse le mélange réactionnel sur une solution aqueuse glacée, de phosphate monosodique, l'extrait au chlorure de méthylène, la lave à l'eau, la sèche, la concentre à sec sous pression réduite, obtient ≃ 221 mg de produit brut qui cristallise, recristallise le produit dans 4 $cm^3$ d'un mélange éther de pétrole – éther isopropylique: 7, 3, obtient 165 mg de produit blanc F≃110°C. Les liqueurs mères chromatographiées donnent 17 mg de cristaux blancs F≃110°C.
$(\alpha)_D = -241°$ (1% benzène)

Spectre de RMN
- pics à 1,1 et 1,13 ppm attribués aux hydrogènes des méthyles géminés
- pic à 2,03 ppm attribué aux hydrogènes en 1 et 5
- pic à 4,96 ppm attribué à l'hydrogène en position 4
- pics à 1,42 et 1,53 ppm attribuées aux hydrogènes du méthyle fixé sur le carbone benzylique
- pics à 4,7–4,8–4,9 et 5,0 ppm attribués à l'hydrogène porté par le carbone benzylique
- pics à 6,83–7,5 ppm attribués aux hydrogènes des noyaux aromatiques

Stade D:

(1R, 5S) 6,6-diméthyl 4-hydroxy 3-oxabicyclo [3.1.0] hexan-2-one

Pendant 2 heures, la 20/25°C, on agite 1 g du produit obtenu précédemment, 10 $cm^3$ d'acétone et 5 $cm^3$ de solution aqueuse normale d'acide chlorhydrique, amène le mélange réactionnel à pH 8 avec du bicarbonate de sodium, le lave au chlorure de méthylène, acidifie la phase aqueuse à pH 1 avec de l'acide chlorhydrique concentré, extrait à l'acétate d'éthyle, sèche et évapore le solvant sous pression réduite. On obtient 384 mg de cristaux F = 117°C.
Produit identique au produit obtenu dans le brevet français n° 1580474 $(\alpha)_D = -110°$ (c = 1% diméthylformamide).

Exemple VIII:

6,6-diméthyl 4-hydroxy 3-oxabicyclo [3.1.0] hexan-2-one dl

Stade A:

4-(2-bromoprop-2-yl) 5-hydroxy tétrahydrofuran-2-one dl trans

La préparation de ce composé est décrite au stade A de l'exemple VI.

Stade B:

4-(2-bromoprop-2-yl) 5-isopropyloxy tétrahydrofuran-2-one dl trans

Pendant 60 heures à température ambiante, on agite 1,95 g du dérivé bromé obtenu précédemment, 2 $cm^3$ d'isopropanol, 100 mg d'acide paratoluènesulfonique et 30 $cm^3$ de benzène, lave le milieu réactionnel par une solution aqueuse de bicarbonate de sodium, extrait au benzène, sèche, concentre à sec sous pression réduite, obtient 2 g de produit huileux utilisé tel quel au stade suivant.

Stade C:

6,6-diméthyl 4-isopropyl 3-oxabicyclo [3.1.0] hexan-2-one dl

On mélange 728 mg du produit obtenu ci-dessus, 5 $cm^3$ de tétrahydrofurane anhydre et 1 $cm^3$ de diméthylsulfoxyde anhydre, refroidit à 0° C, ajoute en une seule fois 150 mg d'hydrure de sodium, après 20 heures d'agitation à température ambiante et sous atmosphère inerte, verse sur une solution aqueuse de phosphate monosodique, extrait au benzène, sèche, concentre à sec sous pression réduite et obtient 450 mg de produit brut, le chromatographie sur silice et élue par le système de solvant éther de pétrole — éther éthylique 7-3, obtient 350 mg du produit attendu.

Spectre de RMN
pics à 1,17—1,31 ppm attribués aux hydrogènes des méthyles en 6 et des méthyles de l'isopropyle
pic à 2,02 ppm attribué aux hydrogènes en 1 et 5 du cycle
pic à 5,25 ppm attribué à l'hydrogène en 4 du cycle
pic à 4,03 ppm attribué à l'hydrogène en 2 du propyle

Stade D:

6,6-diméthyl 4-hydroxy 3-oxabicyclo [3.1.0] hexan-2-one dl

On agite 150 mg du produit obtenu précédemment avec 3 $cm^3$ d'acétone et 1 $cm^3$ d'acide chlorhydrique aqueux N pendant 4 heures à 20°/25° C, sature le mélange réactionnel avec du chlorure de sodium, extrait au chlorure de méthylène, sèche la phase organique, évapore à sec sous pression réduite et obtient 95 mg de cristaux blancs fondant à 80° C.

**Revendications**

1. L'acide 3-formyl 4-méthyl pent-3-èn 1-oïque de formule I

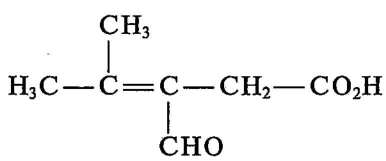

(I)

2. Procédé de préparation de l'acide 3-formyl 3-Méthyl pent-3-èn 1-oïque, de formule I, caractrérisé en ce que l'on fait réagir un dérivé de formule II:

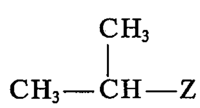

(II)

dans laquelle Z représente un groupement électroattracteur, avec un dérivé de formule III:

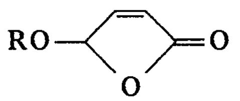

(III)

dans laquelle R représente le reste R d'un alcool ROH, puis soumet le composé résultant de formule IV:

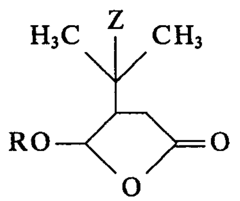

(IV)

à l'action d'un agent basique.

3. Procédé selon la revendication 2, caractérisé en ce que le groupement Z est un groupement $NO_2$ et en ce que l'on fait réagit les composés II et III en présence d'une base choisie dans le groupe constitué par les amines secondaires, les amines tertiaires, les hydroxydes d'ammonium quaternaires et les carbonates alcalins.

4. Procédé selon la revendication 2, caractérisé en ce que le groupement Z est un groupement hydroxyle et en ce que l'on fait réagir les composés II et III sous irradiation ou en présence d'un promoteur radicalaire.

5. Procédé selon la revendication 2, caractérisé en ce que le groupement Z est un groupement aryl sulfonyle et en ce que l'on fait réagir les composés II et III en présence d'une base forte au sein d'un solvant organique.

6. Procédé selon la revendication 2, caractérisé en ce que le groupement Z est un groupement halogéno triaryl phosphonio et en ce que l'on fait réagir les composés II et III en présence d'une base forte au sein d'un solvant organique.

7. Procédé selon la revendication 5 ou 6, caractérisé en ce que la base forte est choisie dans le groupe constitué par les alkyl lithiens, les amidures alcalins, les hydrures alcalins, les alcoolates alcalins et en ce que le solvant utilisé est choisi dans le groupe constitué par le tétrahydrofurane, le diméthylsulfoxyde, le diméthoxyéthane, le diméthylformamide, l'hexaméthylphosphoramide, les hydrocarbures aromatiques et des cycloalcanes.

8. Procédé selon la revendication 2, caractérisé en ce que l'agent basique est le carbonate de sodium utilisé en milieu hydrométhanolique.

9. Application de l'acide 3-formyl 4-méthyl pent-3-èn 1-oïque de formule I à la préparation de composés de formule V:

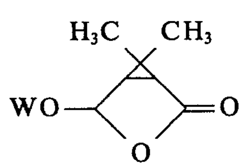

(V)

dans laquelle W représente un atome d'hydrogène ou un reste $R_1$ d'un alcool $R_1OH$ éventuellement chiral, caractérisée en ce que l'on fait réagir en milieu anhydre l'acide 3-formyl 4-méthyl pent-3-èn 1-oïque avec un hydracide HX, dans lequel X représente un atome d'halogène, en présence d'un halogénure de lithium LiX au sein d'un solvant organique, pour obtenir un composé de formule VI:

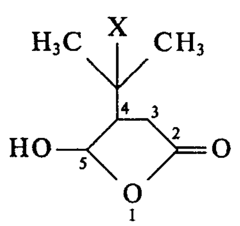

(VI)

que l'on fait réagir soit avec un alcool $R_1OH$ achiral pour obtenir le composé VII racémique:

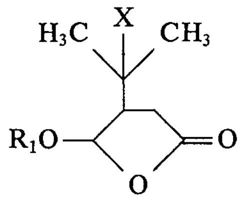

(VII)

qui, comme le dérivé VI, présente une relation trans entre les substituants en 4 et 5 de la tétrahydrofuranone, soit avec un alcool $R_1OH$ chiral, le compose VII étant alors obtenu sous forme d'un mélange des deux diastéréoisomères prévisibles, mélange que l'on peut séparer en ses constituants par des procédés physiques, fait réagir le composé de forme VII, sous forme racémique lorsque $R_1$ est achiral, ou sous forme optiquement active lorsque $R_1$ est chiral, le composé VII étant dans ce dernier cas à l'état de l'un ou de l'autre des diastéréoisomères, avec un agent basique, pour obtenir le composé bicyclique de formule V':

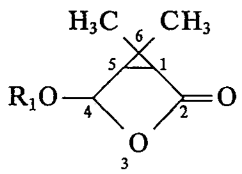

(V')

sous forme racémique ou sous l'une ou l'autre de ses formes énantiomériques selon que $R_1$ est respectivement un reste achiral ou chiral, la configuration absolue en position 4 de ce composé ainsi que celles qui en découlent en position 1 et 5 étant alors déterminées par la stéréochimie du diastéréoisomère VII mis en oeuvre, puis hydrolyse si désiré, l'éther de formule V, en milieu acide, avec rétention totale des configurations absolues, pour obtenir le composé de formule $V_A$ correspondant:

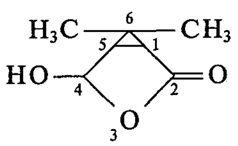

($V_A$)

10. Application selon la revendication 9, caractérisée en ce que la transformation du composé de formule I en composé de formule VI est effectuée à l'aide des couples BrH/BrLi ou ClH/ClLi, au sein de l'éther éthylique anhydre.

11. Application selon la revendication 9, caractérisée en ce que l'on fait réagir l'alcool $R_1OH$, achiral ou chiral et le compose VI en présence d'acide paratoluènesulfonique.

12. Application selon la revendication 9, caractérisée en ce que les deux diastéréoisomères provenant de l'action de l'alcool chiral $R_1OH$ sur le racémate du composé VI, sont séparés par chromatographie ou par cristallisation.

13. Application selon la revendication 9, caractérisée en ce que la transformation du compose VII en composé cyclopropanique V' est effectuée à l'aide de butyllithium.

14. Application selon la revendication 9, caractérisée en ce que la transformation des composés VII en composés cyclopropaniques V' est effectuée par la méthode de catalyse en transfert de phase, à l'aide d'un hydroxyde alcalin, d'eau, d'un solvant non miscible à l'eau et d'un ammonium quaternaire.

15. Application selon la revendication 9, caractérisée en ce que la transformation des composés VII en composés cyclopropaniques V' est effectuée à l'aide d'hydrure de sodium.

16. Application selon la revendication 9, caractérisée en ce que l'hydrolyse du compose V' en composé $V_A$ est effectuée en présence d'acide chlorhydrique, le cas échéant en milieu hydroacétonique.

**Patentansprüche**

1. 3-Formyl-4-methyl-pent-3-en-1-säure der Formel I

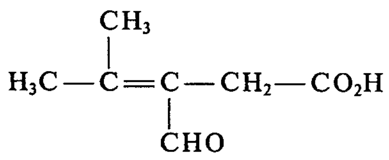

(I)

2. Verfahren zur Herstellung der 3-Formyl-4-methyl-pent-3-en-1-säure der Formel I, dadurch gekennzeichnet, daß man ein Derivat der Formel II

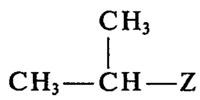

(II)

worin Z eine elektronenanziehende Gruppe darstellt, mit einem Derivat der Formel III

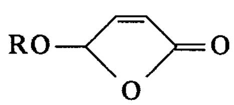

(III)

worin R den Rest R eines Alkohols ROH bedeutet, umsetzt und danach die erhaltene Verbindung der Formel IV

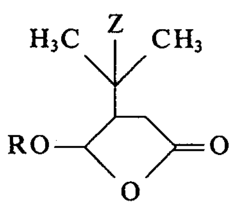

(IV)

der Einwirkung eines basischen Mittels unterzieht.

3. Verfahren gemäß Anspruch 2, dadurch gekennzeichnet, daß die Gruppe Z eine Gruppe $NO_2$ ist und daß man die Verbindung II und III in Gegenwart einer Base, ausgewählt unter den sekundären Aminen, den tertiären Aminen, den quaternären Ammoniumhydroxiden und den Alkalicarbonaten umsetzt.

4. Verfahren gemäß Anspruch 2, dadurch gekennzeichnet, daß die Gruppe Z eine Hydroxylgruppe ist und daß man die Verbindungen II und III unter Bestrahlung oder in Gegenwart eines radikalischen Promotors umsetzt.

5. Verfahren gemäß Anspruch 2, dadurch gekennzeichnet, daß die Gruppe Z eine Arylsulfonylgruppe ist und daß man die Verbindungen II und III in Gegenwart einer starken Base in dem Medium eines organischen Lösungsmittels umsetzt.

6. Verfahren gemäß Anspruch 2, dadurch gekennzeichnet, daß die Gruppe Z eine Halogenotriarylphosphoniogruppe ist und daß man die Verbindungen II und III in Gegenwart einer starken Base in dem Medium eines organischen Lösungsmittels umsetzt.

7. Verfahren gemäß Anspruch 5 oder 6, dadurch gekennzeichnet, daß die starke Base ausgewählt wird unter den Alkyllithiumverbindungen, den Alkaliamiden, den Alkalihydriden, den Alkalialkoholaten und daß das verwendete Lösungsmittel ausgewählt wird unter Tetrahydrofuran, Dimethylsulfoxid, Dimethoxyäthan, Dimethylformamid, Hexamethylphosphoramid, den aromatischen Kohlenwasserstoffen und den Cycloalkanen.

8. Verfahren gemäß Anspruch 2, dadurch gekennzeichnet, daß das basische Mittel in wäßrig-methanolischem Milieu verwendetes Natriumcarbonat ist.

9. Verwendung der 3-Formyl-4-methyl-pent-3-en-1-säure der Formel I zur Herstellung der Verbindungen der Formel V

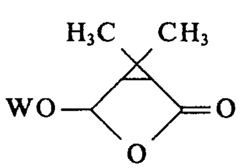

(V)

worin W ein Wasserstoffatom oder einen Rest $R_1$ eines gegebenenfalls chiralen Alkohols $R_1OH$ bedeutet, dadurch gekennzeichnet, daß man in wasserfreiem Milieu die 3-Formyl-4-methyl-pent-3-en-1-säure mit einer Wasserstoffsäure HX, worin X ein Halogenatum bedeutet, in Gegenwart eines Lithiumhalogenids LiX in dem Medium eines organischen Lösungsmittels umsetzt, um eine Verbindung der Formel VI

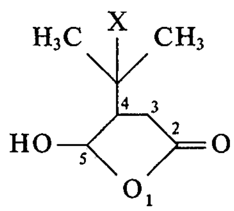

(VI)

zu erhalten, die man entweder mit einem achiralen Alkohol $R_1OH$ umsetzt, um die racemische Verbindung der Formel VII

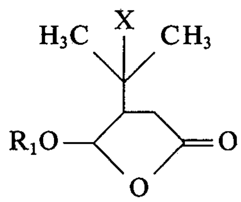

(VII)

zu erhalten, die wie das Derivat VI eine trans-Beziehung zwischen den Substituenten in 4- und 5-Stellung des Tetrahydrofuranons aufweist, oder mit einem chiralen Alkohol $R_1OH$ umsetzt, wobei die Verbindung VII dann in Form eines Gemisches der beiden erwarteten Diastereomeren erhalten wird, welches Gemisch man mit Hilfe physikalischer Verfahren in seine Bestandteile trennen kann, die Verbindung der Formel VII in racemischer Form, wenn $R_1$ achiral ist oder in optisch aktiver Form, wenn $R_1$ chiral ist, wobei die Verbindung VII in diesem letzteren Fall in Form des einen oder des anderen der Diastereomeren vorliegt, mit einem basischen Mittel umsetzt, um die bicyclische Verbindung der Formel V′

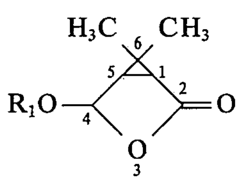

(V′)

in racemischer Form oder in der einen oder der anderen ihrer enantiomeren Formen je nachdem, ob $R_1$ ein achiraler bzw. ein chiraler Rest ist, zu erhalten, wobei die absolute Konfiguration in 4-Stellung dieser Verbindung ebenso wie diejenigen, die von der 1- und 5-Stellung herrühren, dann von der Stereochemie des eingesetzten Diastereomeren VII bestimmt werden, danach gewünschtenfalls den Äther der Formel V′ in saurem Milieu unter vollständiger Beibehaltung der absoluten Konfigurationen hydrolysiert, um die entsprechende Verbindung der Formel $V_A$

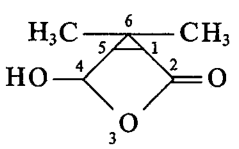

($V_A$)

zu erhalten.

10. Verwendung gemäß Anspruch 9, dadurch gekennzeichnet, daß die Umwandlung der Verbindung der Formel I in die Verbindung der Formel VI mit Hilfe der Paare BrH/BrLi oder ClH/ClLi in wasserfreiem Äthyläthermedium durchgeführt wird.

11. Verwendung gemäß Anspruch 9, dadurch gekennzeichnet, daß man den achiralen oder chiralen Alkohol $R_1OH$ und die Verbindung VI in Gegenwart von para-Toluolsulfonsäure umsetzt.

12. Verwendung gemäß Anspruch 9, dadurch gekennzeichnet, daß die beiden der Umsetzung des chiralen Alkohols $R_1OH$ mit dem Racemat der Verbindung VI entstammenden beiden Diastereomeren durch Chromatographie oder Kristallisation getrennt werden.

13. Verwendung gemäß Anspruch 9, dadurch gekennzeichnet, daß die Umwandlung der Verbindung VII in die Cyclopropanverbindung V′ mit Hilfe von Butyllithium durchgeführt wird.

14. Verwendung gemäß Anspruch 9, dadurch gekennzeichnet, daß die Umwandlung der

Verbindungen VII in die Cyclopropanverbindungen V' nach der Methode der Phasentransferkatalyse mit Hilfe von Alkalihydroxid, Wasser, einem mit Wasser nicht mischbaren Lösungsmittel und quaternärem Ammonium durchgeführt wird.

15. Verwendung gemäß Anspruch 9, dadurch gekennzeichnet, daß die Überführung der Verbindungen VII in die Cyclopropanverbindungen V' mit Hilfe von Natriumhydrid durchgeführt wird.

16. Verwendung gemäß Anspruch 9, dadurch gekennzeichnet, daß die Hydrolyse der Verbindung V' in die Verbindung $V_A$ in Gegenwart von Chlorwasserstoffsäure, gegebenenfalls in wäßrig-acetonischem Milieu durchgeführt wird.

## Claims

1. 3-formyl 4-methyl pent-3-en 1-oic acid with the formula (I):

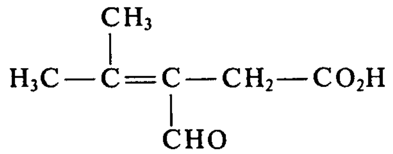

(I)

2. Process for the preparation of 3-formyl 4-methyl pent-3-en 1-oic acid, with the formula (I), characterised in that a derivative with the formula (II):

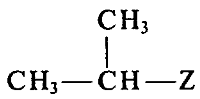

(II)

in which Z represents an electron attracting group, is made to react with a derivative with the formula (III):

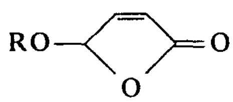

(III)

in which R represents the residue R of an alcohol ROH, then the resultant compound with the formula (IV)

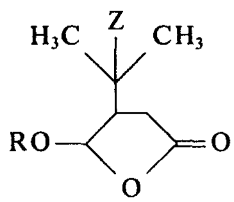

(IV)

is submitted to the action of a basic agent.

3. Process according to Claim 2, characterised in that the group Z is a group $NO_2$ and in that the compound (II) and (III) are made to react in the presence of a base chosen from the group constituted by the secondary amines, the tertiary amines, the quaternary ammonium hydroxides and the alkaline carbonates.

4. Process according to Claim 2, characterised in that the group Z is a hydroxyl group and in that the compounds (II) and (III) are made to react under irradiation or in the presence of a radical promotor.

5. Process according to Claim 2, characterised in that the group Z is an aryl sulphonyl group and in that the compounds (II) and (III) are made to react in the presence of a strong base in an organic solvent.

6. Process according to Claim 2, characterised in that the group Z is a halogen triaryl phosphonic group and in that the compounds (II) and (III) are made to react in the presence of a strong base in an organic solvent.

7. Process according to Claim 5 or 6, characterised in that the strong base is chosen from the group constituted by the lithium alkyls, the alkaline amides, the alkaline hydrides, the alkaline alcoholates, and in that the solvent utilised is chosen from the group constituted by tetrahydrofuran, dimethylsulphoxide, dimethoxyethane, dimethylformamide, hexamethylphosphoramide, the aromatic hydrocarbons and the cycloalkanes.

8. Process according to Claim 2, characterised in that the basic agent is sodium carbonate used in a hydromethanol medium.

9. Application of 3-formyl 4-methyl pent-3-en 1-oic acid with the formula (I) for the preparation of compounds with the formula (V)

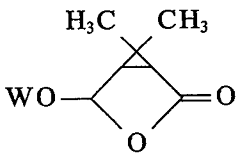

(V)

in which W represents a hydrogen atom or a residue $R_1$ of an alcohol $R_1OH$ possible chiral, characterised n that the 3-formyl 4-methyl pent-3-en 1-oic acid is made to react in an anhydrous medium with a hydracid HX, in which X represents a halogen atom, in the presence of a lithium halide LiX in an organic solvent, so as to obtain a compound with the formula (VI)

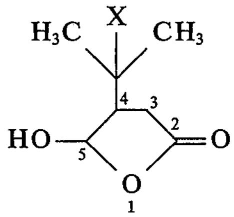

(VI)

which is made to react either with an achiral alcohol $R_1OH$ in order to obtain the racemic compound (VII)

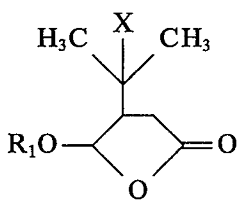

(VII)

which, as in the derivative (VI) presents a trans relation between the substituents in position 4 and 5 of the tetrahydrofuranone, or with a chiral alcohol $R_1OH$ the compound (VII) being then obtained in form of a mixture of the 2 foreseeable diastereo-isomers, which mixture can bei separated into its constituents by physical processes, the compound with the formula (VII) is made to react in racemic form when $R_1$ is achiral, or in the optically active form when $R_1$ is chiral, the compound (VII) being in that latter case in the state of one or other of the diastereo-isomers, with a basic agent, so as to obtain the bicyclic compound with the formula (V′)

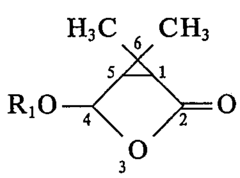

(V′)

in the racemic form or in one or other of its enantiomeric forms according to whether $R_1$ is respectively an achiral or chiral residue, the absolute configuration in position 4 of this compound as well as those which appear in positions 1 and 5 being then determined by the stereo-chemistry of the diastereo-isomer (VII) utilised, then if desired, the ether of formula (V′) is hydrolysed in an acid medium with total retention of the absolute configurations, so as to obtain the corresponding compound with the formula ($V_A$)

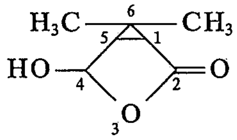

($V_A$)

10. Application according to Claim 9, characterised in that the conversion of the compound with the formula (I) into the compounds with the formula (VI) is carried out by means of the couples BrH/BrLi or ClH/ClLi, in anhydrous ethyl ether.

11. Application according to Claim 9, characterised in that the alcohol $R_1OH$, achiral or chiral is made to react with the compound (VI) in the presence of paratoluenesulphonic acid.

12. Application according to Claim 9, characterised in that the two diastereo-isomers resulting from the action of the chiral alcohol $R_1OH$ on the racemate of Compound (VI) are separated by chromatography or by crystallisation.

13. Application according to Claim 9, characterised in that the conversion of the compound (VII) into the cyclopropane compound (V′) is carried out by means of butyllithium.

14. Application according to Claim 9, characterised in that the conversion of the compound (VII) into cyclopropane compounds (V′) is carried out by the catalytic phase transfer method, by means of an alkaline hydroxide, water, a solvent not miscible with water and a quaternary ammonium.

15. Application according to Claim 9, characterised in that the conversion of the compounds (VII) into cyclopropane compounds (V′) is carried out by means of sodium hydride.

16. Application according to Claim 9, characterised in that the hydrolysis of the compound (V′) into compound ($V_A$) is carried out in the presence of hydrochloric acid, if necessary in a hydroacetone medium.